# EUROPEAN PATENT APPLICATION

(11) **EP 3 875 087 A1**
(43) Date of publication of application: **08.09.2021**
(21) Application number: 19877607.2
(22) Date of filing: 01.11.2019
(51) Int. Cl.: A61K 31/47, A61K 31/426, A61K 31/519, A61P 19/06

(54) **JOINT USE OF COMPOUND A AND COMPOUND B IN PREPARATION OF MEDICATIONS FOR TREATMENT OF GOUT OR HYPERURICEMIA**

(30) Priority: 02.11.2018 CN 201811300282
(71) Applicant: Jiangsu Hengrui Medicine Co., Ltd., Lianyungang, Jiangsu 222047 (CN)
(72) Inventor: CHEN, Jianwen, Lianyungang, Jiangsu 222047 (CN); SHEN, Yang, Lianyungang, Jiangsu 222047 (CN); NING, Rui, Lianyungang, Jiangsu 222047 (CN)
(74) Representative: Regimbeau
(86) International application number: PCT/CN2019/114980
(87) International publication number: WO 2020/088641

(57) **Abstract**

The present disclosure relates to the joint use of compound A and compound B in the preparation of medications for the treatment of gout or hyperuricemia. Specifically, compound A is selected from the compound shown in Formula I, a pharmaceutical salt thereof, or an ester thereof, and compound B is selected from allopurinol or febuxostat. In particular, a method for treating gout or hyperuricemia in which the compound shown in Formula 1-1 works in conjunction with febuxostat shows good treatment effect.

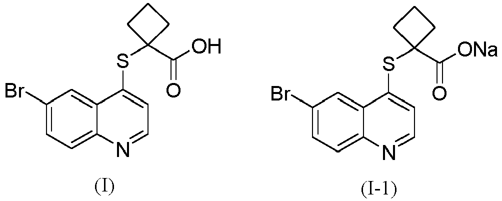

## Description

### FIELD OF THE INVENTION

The present disclosure belongs to the field of medicine, and relates to a use of compound A in combination with compound B selected from the group consisting of febuxostat and allopurinol in the preparation of a medicament for treating gout.

### BACKGROUND OF THE INVENTION

Hyperuricemia (HUA) is a metabolic disease caused by purine metabolism disorder. Hyperuricemia is clinically divided into primary hyperuricemia and secondary hyperuricemia. Primary hyperuricemia is mostly caused by congenital abnormal purine metabolism, and often accompanied by obesity, glucose/lipid metabolism disorder, atherosclerosis, coronary heart disease, hypertension and the like. Secondary hyperuricemia is caused by certain systemic diseases or drugs. When the serum uric acid (sUA) concentration is 6.8 mg/dl, the dissolution of uric acid reaches a saturation state. A concentration beyond this level is defined as hyperuricemia (HUA).

In some patients with hyperuricemia, supersaturated sodium urate precipitates out as microcrystals with the increase of blood uric acid level. The microcrystals are deposited on tissues or organs such as joints, synovium, tendons, kidneys and connective tissues (except the central nervous system) to form gout stones, causing acute and chronic inflammation and tissue damage, resulting in multiple system damages such as arthritis, urinary calculi and kidney disease. The hyperuricemia in about 5% to 12% of patients will eventually develop into gout.

Since the 1980s, the dietary habit of Chinese have changed with the improvement of living standards, and the prevalence of HUA has increased significantly, reaching 5 to 23.5% in coastal and developed areas. Moreover, HUA is tending to occur in younger people. In addition to acute attacks, other symptoms of gout include bone erosion and the formation of subcutaneous gout nodules. A number of studies have confirmed that long-term continuous reduction of serum uric acid (sUA) can reduce the number of gout attacks and gout nodules, and make the gout nodules shrink.

Febuxostat belongs to xanthine oxidase inhibitors (XOIs), which is a commonly used drug for treating gout. The average range of the terminal elimination phase half-life of febuxostat in steady state is 5 to 6 hours, and the steady state can be substantially reached after one administration. However, 40% to 70% of patients using febuxostat still cannot reach the clinically recommended sUA level. Regarding to patients using XOIs treatment that cannot reach the recommended sUA level, the American Rheumatism Association recommends a combined treatment with uric acid excretion drugs.

Allopurinol can inhibit xanthine oxidase, so that hypoxanthine and xanthine cannot be converted into uric acid (*i.e.* uric acid synthesis is reduced), thereby reducing the uric acid concentration in blood and the deposition of urate in bones, joints and kidneys.

The phase III clinical trial data of the combination administration of lesinurad and febuxostat (CRYSTAL) (ARTHRITIS & RHEUMATOLOGY. Vol. 69, No. 9, September 2017, pp 1903-1913) demonstrate that the proportion of patients who achieved sUA<5.0 mg/dl after 6 months was 46.8% in the febuxostat alone group, 56.6% in the lesinurad 200 mg plus febuxostat group, and 76.1% in the lesinurad 400 mg plus febuxostat group. There are significantly more patients reached the standard in the lesinurad 400 mg group (P<0.0001), while the difference was not statistically significant between the lesinurad 200 mg group and the febuxostat alone group (P=0.13). However, the incidence of TEAE increased in the lesinurad 400 mg plus febuxostat group compared with the febuxostat alone group. Compared with the febuxostat alone group, the incidence of kidney-related adverse events in the lesinurad 400 mg plus febuxostat group nearly doubled (5.5% v.s. 10.1%), and the serum creatinine increased ≥ 1.5 times the baseline to 10.1% (2.8% v.s. 10.1%), the serum creatinine increased ≥ 2 times the baseline to 5.5% (0% v.s. 5.5%).

CN104470898A discloses a compound of formula (I) and a pharmaceutically acceptable salt and ester thereof, which is a highly selective URAT1 inhibitor and can increase uric acid excretion by inhibiting URAT1 to significantly reduce serum uric acid (sUA) level.

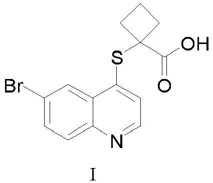

### SUMMARY OF THE INVENTION

The present disclosure provides a method for treating gout or hyperuricemia by the combination of compound A and compound B, which shows a good therapeutic effect and slight adverse reaction (easy to recover/solve). The compound A is selected from the group consisting of the compound of formula (I), the pharmaceutically acceptable salt and ester thereof, and the compound B is selected from the group consisting of allopurinol and febuxostat. The compound A and compound B have a synergistic effect on reducing uric acid. The compound A is preferably a compound of formula (I-1). The compound B is preferably febuxostat.

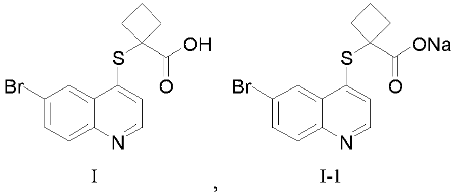

The pharmaceutically acceptable salt can be a hydrochloride, sulfate, citrate, benzenesulfonate, hydrobromide, hydrofluoride, phosphate, acetate, propionate, succinate, oxalate, malate, succinate, fumarate, maleate, tartrate or trifluoroacetate, and can also be an alkali metal ion salt of the compound such as sodium salt and potassium salt. The ester can be a C₁₋₇ straight chain alkyl ester, C₃₋₇ branched chain alkyl ester, C₁₋₄ haloalkyl ester, C₃₋₇ cycloalkyl ester, aryl ester, heteroaryl ester and the like.

The present disclosure provides a use of compound A in combination with compound B in the preparation of a medicament for treating gout or hyperuricemia. The compound A and compound B have a synergistic effect on reducing uric acid. The compound A is selected from the group consisting of the compound of formula (I), the pharmaceutically acceptable salt and ester thereof, and the compound B is selected from the group consisting of allopurinol and febuxostat. The compound A is preferably a compound of formula (I-1). The compound B is preferably febuxostat.

In some embodiments, the serum uric acid level in the subject reduces to below 6 mg/dl after the administration of compound A and compound B.

In some embodiments, the weight ratio of compound A to febuxostat is 0.01-100:1, preferably 1:20, 1:19, 1:18, 1:17, 1:16, 1:15, 1:14, 1:13, 1:12, 1:11, 1:10, 1:9, 1:8, 2:15, 1:7, 1:6, 1:5, 5:24, 2:9, 1:4, 4:15, 5:18, 2:7, 3:10, 5:16, 1:3, 5:14, 3:8, 2:5, 5:12, 3:7, 4:9 or 1:2, and more preferably 1:16, 1:8, 1:4 or 1:2.

In some embodiments, the weight ratio of compound A to allopurinol is 0.01-100:2, preferably 0.01-1:2, and more preferably 1:180, 1:170, 1:160, 1:150, 1:140, 1:130, 1:120, 1:110, 1:100, 1:90, 1:80, 1:70, 1:60, 1:50, 1:40, 1:30, 1:20, 1:10 or 1:5.

In some embodiments, the the dose of compound A is 1 to 100 mg, preferably 1 to 50 mg, more preferably 1 to 20 mg, and most preferably about 5 mg, about 6 mg, about 7 mg, about 8 mg, about 9 mg, about 10 mg, about 11 mg, about 12 mg, about 13 mg, about 14 mg, about 15 mg, about 16 mg, about 17 mg, about 18 mg, about 19 mg or about 20 mg.

In some embodiments, the the dose of febuxostat is 1 to 200 mg, preferably 10 to 120 mg, and more preferably about 10 mg, about 20 mg, about 30 mg, about 40 mg, about 50 mg, about 60 mg, about 70 mg, about 80 mg, about 90 mg, about 100 mg, about 110 mg or about 120 mg.

In some embodiments, the dose of allopurinol is 50 to 900 mg, preferably 100 to 600 mg, and more preferably 100 mg, 150 mg, 200 mg, 250 mg, 300 mg, 350 mg, 400 mg, 450 mg, 500 mg, 550 mg or 600 mg.

The administration route is selected from the group consisting of oral administration, parenteral administration and transdermal administration, and preferably oral administration, the parenteral administration includes but is not limited to intravenous injection, subcutaneous injection, intramuscular injection. In some embodiments, both compound A and compound B are administered orally. In some embodiments, both compound A and compound B are administered in the form of tablets.

The administration frequency of compound A can be once a day, twice a day, once every two days, or once every three days. The administration frequency of compound B can be once a day, twice a day, three times a day, once every two days, or once every three days. In some embodiments, compound A is orally administered once a day, and compound B is administered once a day. In some embodiments, the compound of formula (I-1) is orally administered once a day, and febuxostat is orally administered once a day. In some embodiments, the compound of formula (I-1) is orally administered once a day in an amount of 5 mg or 10 mg, and febuxostat is orally administered once a day in an amount of 20 mg, 40 mg or 80 mg. In a preferred embodiment of the present disclosure, the compound of formula (I-1) is orally administered once a day in an amount of 5 mg or 10 mg, and allopurinol is orally administered once, twice or three times a day in an amount of 50 mg, 100 mg, 150 mg, 200 g, 250 mg, 300 mg, 350 mg, 400 mg, 450 mg, 500 mg, 550 mg, 600 mg, 650 mg, 700 mg, 750 mg, 800 mg, 850 mg, or 900 mg.

In some embodiments, the method of the present disclosure comprises a step of administering any appropriate amount (effective amount) of compound A to an individual in need thereof. In some embodiments, about 1 to 100 mg of compound A is administered. In some embodiments, about 1 to 50 mg of compound A is administered. In some embodiments, about 1 to 20 mg of compound A is administered. In some embodiments, about 5 mg, about 6 mg, about 7 mg, about 8 mg, about 9 mg, about 10 mg, about 11 mg, about 12 mg, about 13 mg, about 14 mg, about 15 mg, about 16 mg, about 17 mg, about 18 mg, about 19 mg or about 20 mg of compound A is administered.

In some embodiments, the method of the present disclosure comprises a step of administering to an individual in need thereof any appropriate amount (effective amount) of compound B selected from the group consisting of febuxostat and allopurinol. In some embodiments, about 1 to 200 mg, preferably 10 to 120 mg, and more preferably about 10 mg, about 20 mg, about 30 mg, about 40 mg, about 50 mg, about 60 mg, about 70 mg, about 80 mg, about 90 mg, about 100 mg, about 110 mg, or about 120 mg of febuxostat is administered. In some embodiments, about 50 to 900 mg, preferably about 100 to 600 mg, and more preferably about 100 mg, 150 mg, 200 mg, 250 mg, 300 mg, 350 mg, 400 mg, 450 mg, 500 mg, 550 mg or about 600 mg of allopurinol is administered.

In some embodiments, about 1 to 20 mg of compound A and about 20 mg of febuxostat are administered. In some embodiments, about 1 to 20 mg of compound A and about 40 mg of febuxostat are administered. In some embodiments, about 1 to 20 mg of compound A and about 80 mg of febuxostat are administered.

In some embodiments, about 1 to 20 mg of compound A and about 50 to 900 mg of allopurinol are administered. In some embodiments, about 1 to 20 mg of compound A and about 100 to 600 mg of allopurinol are administered. In some embodiments, about 1 to 20 mg of compound A and about 100 mg, 150 mg, 200 mg, 250 mg, 300 mg, 350 mg, 400 mg, 450 mg, 500 mg, 550 mg or about 600 mg of allopurinol are administered.

The present disclosure also provides a method for reducing the uric acid level in one or more tissues, joints, organs or blood of a patient with elevated uric acid level (such as a patient with hyperuricemia or gout) by administering a therapeutically effective amount of compound A.

The present disclosure provides a method for reducing the uric acid level in one or more tissues, joints, organs or blood of a patient who needs to reduce the uric acid level, comprising a step of administering compound A and compound B to the patient. The compound A and compound B have a synergistic effect on reducing uric acid. In some embodiments, compound A and compound B are administered simultaneously. In some embodiments, compound A and compound B are administered at different times. The compound A is selected from the group consisting of the compound of formula (I), the pharmaceutically acceptable salt and ester thereof, and the compound B is selected from the group consisting of allopurinol and febuxostat. The compound A is preferably a compound of formula (I-1). The compound B is preferably febuxostat. In some embodiments, about 10 to 120 mg of febuxostat is administered. In some embodiments, about 20 mg of febuxostat is administered. In some embodiments, about 40 mg of febuxostat is administered. In some embodiments, about 80 mg of febuxostat is administered.

The present disclosure also provides a method for reducing the uric acid level in one or more tissues, joints, organs or blood of a patient who needs to reduce the uric acid level, comprising a step of administering febuxostat and the compound of formula (I-1) to the patient. The febuxostat and the compound of formula (I-1) have a synergistic effect on reducing uric acid. In some embodiments, febuxostat and the compound of formula (I-1) are administered simultaneously. In some embodiments, febuxostat and the compound of formula (I-1) are administered at different times. In some embodiments, about 10 to 120 mg of febuxostat is administered. In some embodiments, about 20 mg of febuxostat is administered. In some embodiments, about 40 mg of febuxostat is administered. In some embodiments, about 80 mg of febuxostat is administered.

The present disclosure also provides a method for reducing the uric acid level in one or more tissues, joints, organs or blood of a patient who needs to reduce the uric acid level, comprising a step of administering compound A and compound B to the patient. In some embodiments, compound A and compound B are administered simultaneously. In some embodiments, compound A and compound B are administered at different times. In some embodiments, about 1 to 50 mg of compound A is administered. In some embodiments, about 1 to 20 mg of compound A is administered. In some embodiments, about 5 mg, about 6 mg, about 7 mg, about 8 mg, about 9 mg, about 10 mg, about 11 mg, about 12 mg, about 13 mg, about 14 mg, about 15 mg, about 16 mg, about 17 mg, about 18 mg, about 19 mg or about 20 mg of compound A is administered. In some embodiments, about 1 to 20 mg of compound A and about 10 to 120 mg of febuxostat are administered. In some embodiments, about 1 to 20 mg of compound A and about 100 to 600 mg of allopurinol are administered.

The present disclosure also provides a method for reducing the uric acid level in one or more tissues, joints, organs or blood of a patient who needs to reduce the uric acid level, comprising a step of administering febuxostat and the compound of formula (I-1) to the patient. In some embodiments, febuxostat and the compound of formula (I-1) are administered simultaneously. In some embodiments, febuxostat and the compound of formula (I-1) are administered at different times. In some embodiments, about 1 to 50 mg of the compound of formula (I-1) is administered. In some embodiments, about 1 to 20 mg of the compound of formula (I-1) is administered. In some embodiments, about 5 mg, about 6 mg, about 7 mg, about 8 mg, about 9 mg, about 10 mg, about 11 mg, about 12 mg, about 13 mg, about 14 mg, about 15 mg, about 16 mg, about 17 mg, about 18 mg, about 19 mg or about 20 mg of the compound of formula (I-1) is administered. In some embodiments, about 1 to 20 mg of the compound of formula (I-1) and about 10 to 120 mg of febuxostat are administered.

The present disclosure also provides a method for reducing the uric acid level in one or more tissues, joints, organs or blood of a patient who needs to reduce the uric acid level, comprising a step of administering allopurinol and the compound of formula (I-1) to the patient. In some embodiments, allopurinol and the compound of formula (I-1) are administered simultaneously. In some embodiments, allopurinol and the compound of formula (I-1) are administered at different times. In some embodiments, about 1 to 50 mg of the compound of formula (I-1) is administered. In some embodiments, about 1 to 20 mg of the compound of formula (I-1) is administered. In some embodiments, about 5 mg, about 6 mg, about 7 mg, about 8 mg, about 9 mg, about 10 mg, about 11 mg, about 12 mg, about 13 mg, about 14 mg, about 15 mg, about 16 mg, about 17 mg, about 18 mg, about 19 mg or about 20 mg of the compound of formula (I-1) is administered. In some embodiments, about 1 to 20 mg of the compound of formula (I-1) and about 100 to 600 mg of allopurinol are administered.

In some embodiments, about 1 to 50 mg of compound A is administered. In some embodiments, about 1 to 20 mg of compound A is administered. In some embodiments, the patient treated according to any method described in the present disclosure is suffered from a disease characterized by abnormally high uric acid content in one or more tissues or organs of the patient. In some embodiments, the disease is characterized by overproduction of uric acid, low uric acid excretion, tumor lysis, blood disorders, or a combination thereof. In some embodiments, the patient who needs to reduce serum uric acid level and/or the patient described in the present disclosure is suffered from gout, recurrent gout attacks, gouty arthritis, hyperuricemia, hypertension, cardiovascular disease, coronary heart disease, Lesch-Nain syndrome, Kelley-Seegmiller syndrome, nephropathy, kidney stones, renal failure, joint inflammation, arthritis, urinary calculi, lead poisoning, hyperparathyroidism, psoriasis or sarcoidosis. In some embodiments, the uric acid level of the individual receiving the treatment described in the present disclosure is reduced by at least about 10% (or >10%) after such treatment. In some embodiments, the uric acid level is reduced by at least about 25% (or >25%). In some embodiments, the uric acid level is reduced by at least about 50% (or >50%). In some embodiments, the tissue or organ is blood.

In some embodiments, the blood uric acid level of the individual receiving the treatment described in the present disclosure is reduced by at least about 1 mg/dl after such treatment. In some embodiments, the blood uric acid level is reduced by at least about 1.5 mg/dl. In some embodiments, the blood uric acid level is reduced by at least about 2 mg/dl. In some embodiments, the blood uric acid level is reduced by at least about 2.5 mg/dl. In some embodiments, the blood uric acid level is reduced by at least about 3 mg/dl. In some embodiments, the blood uric acid level is reduced by at least about 3.5 mg/dl. In some embodiments, the blood uric acid level is reduced by at least about 4 mg/dl. In some embodiments, the blood uric acid level is reduced by at least about 4.5 mg/dl. In some embodiments, the blood uric acid level is reduced by at least about 5 mg/dl. In some embodiments, the blood uric acid level is reduced by at least about 5.5 mg/dl. In some embodiments, the blood uric acid level is reduced by at least about 6 mg/dl. In some embodiments, the blood uric acid level is reduced by more than about 6 mg/dl. The blood uric acid level used in the present disclosure can refer to the uric acid level observed in whole blood or components thereof, such as serum. The disclosure of the serum uric acid level in the present disclosure should be understood as the disclosure describing the blood uric acid level.

In some embodiments, the blood uric acid level of the individual receiving the treatment described in the present disclosure is reduced to at least about 7 mg/dl (*i.e.,* reduced to 7 mg/dl or less) after such treatment. In some embodiments, the blood uric acid level is reduced to at least about 6.5 mg/dl. In some embodiments, the blood uric acid level is reduced to at least about 6 mg/dl. In some embodiments, the blood uric acid level is reduced to at least about 5.5 mg/dl. In some embodiments, the blood uric acid level is reduced to at least about 5 mg/dl. In some embodiments, the blood uric acid level is reduced to at least about 4.5 mg/dl. In some embodiments, the blood uric acid level is reduced to at least about 4 mg/dl.

The present disclosure also provides a method for synergistically reducing the serum uric acid level, comprising a step of administering compound A and compound B to a patient. The compound A is selected from the group consisting of the compound of formula (I), the pharmaceutically acceptable salt and ester thereof, and the compound B is selected from the group consisting of allopurinol and febuxostat. The compound A is preferably a compound of formula (I-1). The compound B is preferably febuxostat.

The present disclosure also describes a method for reducing the serum uric acid level of a patient, comprising a step of administering compound A to the subject, wherein the patient has a serum uric acid level higher than 6.0 mg/dl before the administration, and the patient has a reduced serum uric acid level below about 6.0 mg/dl after the administration. In some embodiments, the patient has a serum uric acid level higher than 6.5 mg/dl, higher than 7.0 mg/dl, higher than 7.5 mg/dl, or higher than 8.0 mg/dl or more before the treatment. The compound A is selected from the group consisting of the compound of formula (I), the pharmaceutically acceptable salt and ester thereof, and preferably the compound of formula (I-1). In some embodiments, the method of the present disclosure further comprises a step of administering compound B selected from the group consisting of febuxostat and allopurinol, and the compound B is preferably febuxostat.

Compound A is administered in any appropriate amount in the methods described in the present disclosure. In some embodiments, about 1 to 50 mg of compound A is administered. In some embodiments, about 1 to 20 mg of compound A is administered. In some embodiments, compound A is administered once a day. In some embodiments, compound A is administered more than once a day. In some embodiments, compound A is administered twice a day. The compound A is selected from the group consisting of the compound of formula (I), the pharmaceutically acceptable salt and ester thereof, and preferably the compound of formula (I-1).

In some embodiments, the method for treating or preventing hyperuricemia or gout comprises a step of administering about 10 to 120 mg of febuxostat and the compound of formula (I-1). In some embodiments, the patient has received a febuxostat treatment and the febuxostat treatment did not reduce the serum uric acid level to below about 6 mg/ml, and the serum uric acid level is reduced to below 6 mg/dl after the administration of febuxostat and the compound of formula (I-1).

The present disclosure also provides a pharmaceutical composition comprising compound A, compound B and at least one pharmaceutically acceptable carrier. The compound A is selected from the group consisting of the compound of formula (I), the pharmaceutically acceptable salt and ester thereof, and preferably the compound of formula (I-1). The compound B is selected from the group consisting of febuxostat and allopurinol. In some embodiments, the composition comprises about 1 to 50 mg of compound A. In some embodiments, the composition comprises about 1 to 20 mg of compound A. In some embodiments, the composition comprises about 5 mg, about 6 mg, about 7 mg, about 8 mg, about 9 mg, about 10 mg, about 11 mg, about 12 mg, about 13 mg, about 14 mg, about 15 mg, about 16 mg, about 17 mg, about 18 mg, about 19 mg or about 20 mg of compound A. In some embodiments, the composition comprises about 10 to 120 mg of febuxostat. In some embodiments, the composition comprises about 10 mg, about 20 mg, about 30 mg, about 40 mg, about 50 mg, about 60 mg, about 70 mg, about 80 mg, about 90 mg, about 100 mg, about 110 mg, or about 120 mg of febuxostat. In some embodiments, the composition comprises about 50 to 900 mg of allopurinol. In certain embodiments, the composition comprises about 100 to 600 mg of allopurinol. In some embodiments, the composition comprises about 100 mg, 150 mg, 200 mg, 250 mg, 300 mg, 350 mg, 400 mg, 450 mg, 500 mg, 550 mg or about 600 mg of allopurinol. The compound A is preferably a compound of formula (I-1). The compound B is preferably febuxostat.

When compound A and compound B of the present disclosure are administered in combination, they have a synergistic effect. When compound A and compound B of the present disclosure are administered in combination for treating gout or hyperuricemia, they have a synergistic effect. When compound A and compound B of the present disclosure are administered in combination, they have a synergistic effect on reducing serum uric acid. When compound A and compound B of the present disclosure are administered in combination, they have a synergistic effect on reducing serum uric acid, the sUA concentration reduces to a certain degree after the administration compared with compound A alone, indicating a synergistic uric acid reducing effect on pharmacodynamics. When compound A and compound B of the present disclosure are administered in combination, they have a synergistic effect on reducing serum uric acid, the sUA concentration reduces to a certain degree after the administration compared with compound A alone, and the maximum reduction is 30-70%. The compound A is selected from the group consisting of the compound of formula (I), the pharmaceutically acceptable salt and ester thereof, and preferably the compound of formula (I-1). The compound B is selected from the group consisting of febuxostat and allopurinol, and preferably febuxostat.

### DEFINITION

In the present disclosure, compound A or compound B refers to compound A or compound B itself, or a pharmaceutical composition comprising compound A or compound B. The compound of formula (I), or the pharmaceutically acceptable salt thereof, or the ester thereof, or the compound of formula (I-1) refers to the compound itself, or a pharmaceutical composition comprising the compound in a pharmaceutically acceptable form. Febuxostat or allopurinol refers to febuxostat or allopurinol itself, or a pharmaceutical composition comprising any pharmaceutically acceptable form of febuxostat or allopurinol.

In the present disclosure, the term "combination" is an administration mode, which refers to the administration of at least one dose of compound A and at least one dose of compound B within a certain period of time, wherein both substances show pharmacological effects. The period of time can be within one administration cycle, within 1 week, or within 24 hours, and more preferably within 12 hours. Compound A and compound B can be administered simultaneously or sequentially. The period of time includes a treatment in which compound A and compound B are administered by the same administration route or different administration routes. The administration mode of the combination of the present disclosure is selected from the group consisting of simultaneous administration, co-administration after separate formulation, and sequential administration after separate formulation.

The combination of the present disclosure can refer to the simultaneous administration of compound A and compound B, the term "simultaneous" used in the present disclosure means that the administration times of compound A and compound B are at least partially overlapped. Therefore, the simultaneous administration includes a dosing regimen in which the administration of one drug is continued after the administration of another drug is stopped.

The term "effective amount" refers to the amount of a drug effective to treat a disease or condition in a mammal.

In the present disclosure, unless otherwise specified, the dose refers to the daily dose.

In the present disclosure, the term "about" means that an embodiment that includes an error of ±5%.

Conversion of uric acid unit mg/dl and µmol/l: mg/dl × 59.5=µmol/l; µmol/l × 0.0168= mg/dl.

### DESCRIPTION OF THE DRAWINGS

Figure 1: The sUA concentration and percentage change of sUA concentration relative to baseline (Mean±SD) after the separate administration and combined administration of colchicine, febuxostat and the compound of formula (I-1);
Figure 2: The percentage change of sUA relative to baseline (D3-0 h) (Mean±SD) after the multiple sequential administration of the compound of formula (I-1) and combined administration of the compound of formula (I-1) and febuxostat.

### DETAILED DESCRIPTION OF THE INVENTION

The present disclosure will be further described with reference to the following examples, but the examples should not be considered as limiting the scope of the present disclosure.

### Example 1: Study of the drug interaction of the compound of formula (I-1), febuxostat and colchicine in patients suffering from gout (single center, single arm, open, self-control)

### Test drugs:

Tablets of the compound of formula (I-1): 5 mg/tablet, provided by Jiangsu Hengrui Medicine Co. Ltd.;
Febuxostat tablets (Ruiyang®): 80 mg/tablet, provided by Jiangsu Hengrui Medicine Co. Ltd.;
Colchicine tablets: 0.5 mg/tablet, provided by Jiangsu Hengrui Medicine Co. Ltd.

### Study objective:

**Main objective:** To evaluate the pharmacokinetic (PK) interaction of the compound of formula (I-1), febuxostat and colchicine adminisered orally in patients suffering from gout.
**Secondary objective:** To evaluate the safety, tolerance and pharmacodynamic (PD) interaction of the compound of formula (I-1), febuxostat and colchicine adminisered orally in patients suffering from gout.

### Study process:

The subjects were screened and examined on D-21 to D-15. 0.5 mg of colchicine once a day (qd) were orally administered to those who passed the screening on an empty stomach (D-14 to D16). If the subject was receiving an uric acid-lowering therapy (including allopurinol, febuxostat, benzbromarone, probenecid and the like) at the time of screening, the drug should be withdrawn for at least 2 weeks before D1. The subjects were admitted to the phase I clinical trial ward on D-2, and medical history inquiry, physical examination, vital signs examination, 12-lead electrocardiogram (ECG) examination, blood pregnancy examination (for female of childbearing age) were conducted again [if D-2 was more than 20 days away from the screening period, then blood examination, urine routine examination, CK\CK-MB, blood biochemistry (including serum creatinine and uric acid) examination were also required].

During the hospital stay, the subjects were fasted for more than 10 hours after dinner everyday (with free access to water). On D3 to D6, the drug together with 240 ml of water were orally adminisered to the subjects on an empty stomach in the morning, and the subjects had a standard meal 1 hour later. On D-1, D1, D7 and D8, the drug together with 240 ml of water were orally adminisered to the subjects on an empty stomach in the morning, and the subjects had a standard meal 4 hours later. On D2, the subjects on an empty stomach were subjected to blood sampling, followed by having a standard meal which should be eaten within 20 minutes. Except for before the drug administration and 1 hour after the drug administration, water was drunk as needed at other times. On D3 to 8, no less than 2 L of water was drunk daily. On D9, the subjects can be discharged from the hospital after completing relevant examinations and operations, with a total hospital stay of 10 days. On D16 to 23, the subjects came back to the phase I clinical trial ward for follow-up. Throughout the study period, the subjects should avoid strenuous exercise and prolonged bed rest. The drug administration scheme is detailed in Table 1.

**Table 1. Drug administration scheme**

| D-21 to D-1 | | D1 | D2 | D3 to D7 | D8 | D9 | D10 to D23 |
|---|---|---|---|---|---|---|---|
| | | | | | | | |
| | Colchicine 0.5 mg qd (D-14 to D16) | | | | | | |
| | | | | | | | |
| Screening period | Introduction period | Febuxostat 80 mg qd | Observation period | Compound of formula (I-1) 10 mg qd | Febuxostat 80 mg qd + compound of formula (I-1) 10 mg qd | Observation period | Follow-up |
| D-21 to D-15 | D-14 to D-1 | | | | | | |

Colchicine tablets: D-14 to D16, 0.5 mg qd, orally adminisered on an empty stomach;
Febuxostat tablets: D1 and D8, 80 mg qd, orally adminisered on an empty stomach;
Tablets of the compound of formula (I-1): D3 to D8, 10 mg qd, orally adminisered on an empty stomach.

### Inclusion criteria:

1. Males and non-lactating and non-pregnant females aged between 18 and 65 (including both ends);
2. The subject was diagnosed as gout according to the classification criteria for acute primary gouty arthritis of American College of Rheumatology (the subject met any of the following 3 items):
   1) Tophi contained uric acid crystals;
   2) Synovial fluid contained uric acid crystals;
   3) The medical history met at least 6 items of the following criteria:
      i. At least one attack of acute arthritis;
      ii. Inflammation reached its severest within 1 day;
      iii. Monoarthritis;
      iv. Redness of joints;
      v. Pain or swelling of the first toe joint;
      vi. Gout attack in one side of the first toe joint;
      vii. Gout attack in one side of the tarsal joint;
      viii. Tophi;
      ix. Hyperuricemia;
      x. X-ray examination showed joint swelling on one side;
      xi. X-ray examination showed cyst under bone cortex without erosion;
      xii. Microbial culture of the joint cavity fluid was negative during the gout attack;
3. Body mass index (weight/height squared) is between 18.5 and 30 (including both ends);
4. 8 mg/dl (480 µmol/l) ≤ sUA during the screening ≤ 10 mg/dl (600 µmol/l).

### Main observation indicator

PK parameters of the compound of formula (I-1), febuxostat (Ruiyang®) and colchicine in plasma.

### Secondary observation indicators

1. sUA level;
2. Safety indicators: any adverse events (including gout attack), physical examination, vital signs (heart rate, respiration, blood pressure, body temperature), laboratory examinations (blood routine examination, urine routine examination, blood biochemistry examination, CK/CK-MB), 12-lead ECG examination and the like.

### Test results:

**Table 2. PK interaction of febuxostat or the compound of formula (I-1) and colchicine**

| Interaction | Parameters (unit) | Geometric mean | | | | |
|---|---|---|---|---|---|---|
| | | Single administration | Combined administration | Ratio (combined administration vs single administration)% | 90%CI | *P*-Value |
| Impact of febuxostat on colchicine | AUCₛₛ (h*ng/mL) | 18.4329 | 18.3270 | 99.43 | 83.34, 118.61 | 0.956 |
| | C_{ss, max} (ng/mL) | 2.5089 | 2.2601 | 90.08 | 70.60, 114.95 | 0.471 |
| Impact of compound of formula (1-1) on colchicine | AUCₛₛ (h*ng/mL) | 18.4329 | 16.6199 | 90.16 | 76.13, 106.79 | 0.306 |
| | C_{ss, max} (ng/mL) | 2.5089 | 2.2048 | 87.88 | 70.30, 109.85 | 0.332 |

**Table 3. PK interaction between febuxostat and the compound of formula (I-1)**

| Interaction | Parameters (unit) | Geometric mean | | | | |
|---|---|---|---|---|---|---|
| | | Single administration | Combined administration | Ratio (combined administration vs single administration)% | 90%CI | *P*-Value |
| Impact of febuxostat on the compound of formula (I-1) | AUCₛₛ (h*ng/mL) | 14352.10 | 14683.66 | 102.31 | 90.07, 116.21 | 0.762 |
| | C_{ss, max} (ng/mL) | 1659.60 | 1687.45 | 101.68 | 88.76, 116.48 | 0.836 |
| Impact of compound of formula (1-1) on febuxostat | AUC₀₋ₗₐₛₜ (h*ng/mL) | 10446.122 | 9248.523 | 88.54 | 75.33, 104.05 | 0.209 |
| | Cₘₐₓ (ng/mL) | 2790.206 | 2411.394 | 86.42 | 63.86, 116.96 | 0.417 |

The serum uric acid (sUA) concentration and percentage change of sUA concentration relative to baseline after the separate administration and combined administration of febuxostat and the compound of formula (I-1) are shown in Figure 1 and Figure 2.

**Table 4. Analysis of the severity scale of all adverse events in the subjects (SS)**

| Preferred term | N=14 | |
|---|---|---|
| | Number of occurrences (%) | Number of cases |
| **Increase of blood bilirubin (D1 to D2)** | 1(7.14) | 1 |
| Mild | 1(7.14) | 1 |
| Moderate | 0 | 0 |
| Severe | 0 | 0 |

| **Increase of blood creatine phosphokinase (D3 to D7)** | 1(7.14) | 1 |
|---|---|---|
| Mild | 1(7.14) | 1 |
| Moderate | 0 | 0 |
| Severe | 0 | 0 |

| **Upper respiratory tract infection (D-21 to D-1)** | 1(7.14) | 1 |
|---|---|---|
| Mild | 1(7.14) | 1 |
| Moderate | 0 | 0 |
| Severe | 0 | 0 |

| **Gout (D8 to D23)** | 1(7.14) | 1 |
|---|---|---|
| Mild | 1(7.14) | 1 |
| Moderate | 0 | 0 |
| Severe | 0 | 0 |

| **Fever (D3 to D7)** | 1(7.14) | 1 |
|---|---|---|
| Mild | 1(7.14) | 1 |
| Moderate | 0 | 0 |
| Severe | 0 | 0 |

| **Diarrhea (D1 to D2)** | 1(7.14) | 1 |
|---|---|---|
| Mild | 1(7.14) | 1 |
| Moderate | 0 | 0 |
| Severe | 0 | 0 |

### Conclusion:

Pharmacokinetic interaction of the compound of formula (I-1), febuxostat and colchicine: The combined administration of febuxostat and colchicine had little effect on the main PK parameters of colchicine in patients suffering from gout, showing that febuxostat had substantially no effect on the AUCₛₛ of colchicine, but the C_{ss,max} of colchicine slightly decreased by 9.92% with no statistical difference (P>0.05); the combined administration of the compound of formula (I-1) and colchicine caused a decrease of the overall exposure level (AUCₛₛ and C_{ss,max}) of colchicine in patients suffering from gout by 9.84% and 12.12% respectively with no statistical difference (P>0.05). When the patients suffering from gout were subjected to the combined administration of febuxostat and the compound of formula (I-1), febuxostat had substantially no effect on the steady-state exposure level of the compound of formula (I-1), but the compound of formula (I-1) caused a slight decrease of the overall exposure (AUC₀₋ₗₐₛₜ and Cₘₐₓ) of febuxostat, AUC₀₋ₗₐₛₜ and Cₘₐₓ respectively decreased by 11.46% and 13.58%, with no statistical difference (P>0.05).

Pharmacodynamic interaction of the compound of formula (I-1), febuxostat and colchicine: When colchicine reached the steady state, the single administration of febuxostat caused a decrease in the serum uric acid (sUA) concentration compared with the single administration of colchicine, the average maximum reduction was 22.144%; when the compound of formula (I-1) was administered sequentially, the serum sUA concentration decreased continuously relative to the baseline (before the administration of the compound of formula (I-1) (D3-0 h)), the average maximum reduction was 60.883%; when the combination of febuxostat and the compound of formula (I-1) was administered, it had a synergistic effect on the reduction of serum uric acid, the sUA concentration at each time point after the combined administration decreased to a certain extent compared with the administration of the compound of formula (I-1) alone, the average maximum decrease occurred at 12 hours after the combined administration, and the maximum decrease percentage of sUA was 41.171%. The above data show that the combined administration of febuxostat and the compound of formula (I-1) significantly increases the uric acid reducing effect compared with single administration, and has a synergistic uric acid reducing effect on pharmacodynamics, which enables the significant reduce of serum uric acid concentration in patients suffering from gout.

During the entire study, no severe adverse events occurred. A total of 6 patients had mild adverse events, which were recovered/resolved during the study.

## Claims

1. Use of compound A in combination with compound B in the preparation of a medicament for treating gout or hyperuricemia, wherein the compound A is selected from the group consisting of a compound of formula (I), a pharmaceutically acceptable salt and ester thereof, and preferably a compound of formula (I-1), and the compound B is selected from the group consisting of allopurinol and febuxostat,

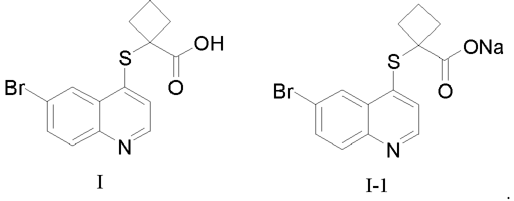

2. The use according to claim 1, **characterized in that** the compound A and compound B have a synergistic effect on reducing uric acid.

3. The use according to claim 1, **characterized in that** the weight ratio of compound A to febuxostat is 0.01-100:1, preferably 1:20, 1:19, 1:18, 1:17, 1:16, 1:15, 1:14, 1:13, 1:12, 1:11, 1:10, 1:9, 1:8, 2:15, 1:7, 1:6, 1:5, 5:24, 2:9, 1:4, 4:15, 5:18, 2:7, 3:10, 5:16, 1:3, 5:14, 3:8, 2:5, 5:12, 3:7, 4:9 or 1:2, and more preferably 1:16, 1:8, 1:4 or 1:2.

4. The use according to claim 1, **characterized in that** the weight ratio of compound A to allopurinol is 0.01-100:2, preferably 0.01-1:2, and more preferably 1:180, 1:170, 1:160, 1:150, 1:140, 1:130, 1:120, 1:110, 1:100, 1:90, 1:80, 1:70, 1:60, 1:50, 1:40, 1:30, 1:20, 1:10 or 1:5.

5. The use according to claim 1, **characterized in that** the the dose of compound A is 1 to 100 mg, preferably 1 to 50 mg, more preferably 1 to 20 mg, and most preferably about 5 mg, about 6 mg, about 7 mg, about 8 mg, about 9 mg, about 10 mg, about 11 mg, about 12 mg, about 13 mg, about 14 mg, about 15 mg, about 16 mg, about 17 mg, about 18 mg, about 19 mg or about 20 mg.

6. The use according to claim 1, **characterized in that** the dose of febuxostat is 1 to 200 mg, preferably 10 to 120 mg, and more preferably about 10 mg, about 20 mg, about 30 mg, about 40 mg, about 50 mg, about 60 mg, about 70 mg, about 80 mg, about 90 mg, about 100 mg, about 110 mg or about 120 mg.

7. The use according to claim 1, **characterized in that** the dose of allopurinol is 50 to 900 mg, preferably 100 to 600 mg, and more preferably 100 mg, 150 mg, 200 mg, 250 mg, 300 mg, 350 mg, 400 mg, 450 mg, 500 mg, 550 mg or 600 mg.

8. The use according to claim 1, **characterized in that** both compound A and compound B are administered orally, and preferably both compound A and compound B are administered in the form of tablets.

9. Use of the compound of formula (I-1) in combination with febuxostat in the preparation of a medicament for treating hyperuricemia or gout.

10. The use according to claim 9, **characterized in that** the dose of the compound of formula (I-1) is 5 mg/qd or 10 mg/qd, and the dose of febuxostat is 20 mg/qd, 40 mg/qd or 80 mg/qd.

11. The use according to claim 9, **characterized in that** the dose of the compound of formula (I-1) is 5 mg/qd or 10 mg/qd, the dose of febuxostat is 20 mg/qd, 40 mg/qd or 80 mg/qd, and both the compound of formula (I-1) and febuxostat are administered once a day in the form of tablets.

12. A pharmaceutical composition, comprising
compound A selected from the group consisting of the compound of formula (I), the pharmaceutically acceptable salt and ester thereof;
compound B selected from the group consisting of febuxostat and allopurinol;
at least one pharmaceutically acceptable carrier;
wherein compound A is preferably the compound of formula (I-1), and compound B is preferably febuxostat.

13. The pharmaceutical composition according to claim 12, **characterized in that** the dose of the compound of formula (I-1) is 5 mg or 10 mg, the dose of febuxostat is 20 mg, 40 mg or 80 mg, and the dose of allopurinol is 100 to 600 mg.
